# EUROPÄISCHE PATENTSCHRIFT

(11) **EP 0 472 556 B1**
(45) Veröffentlichungstag und Bekanntmachung des Hinweises auf die Patenterteilung: **02.11.1994**
(21) Anmeldenummer: 90906997.3
(22) Anmeldetag: 11.05.1990
(51) Int. Cl.: C07H 7/033, C07H 13/04

(54) **VERFAHREN ZUR HERSTELLUNG VON 1-FLUOR-GLYCORONSÄUREN UND DEREN SALZEN SOWIE SOLCHE NEUEN 1-FLUOR-GLYCURONSÄUREN UND DEREN SALZE**
PROCESS FOR PRODUCING 1-FLUORO-GLYCURONIC ACIDS AND THEIR SALTS AND SUCH NEW 1-FLUORO-GLYCONIC ACIDS AND THEIR SALTS
PROCEDE DE PRODUCTION D'ACIDES 1-FLUOROGLYCURONIQUES ET LEURS SELS, CES NOUVEAUX ACIDES 1-FLUOROGLYCURONIQUES ET LEURS SELS

(30) Priorität: 18.05.1989 DE 3916206
(43) Veröffentlichungstag der Anmeldung: 04.03.1992
(73) Patentinhaber: HOECHST AKTIENGESELLSCHAFT, 65926 Frankfurt am Main (DE)
(72) Erfinder: LEUPOLD, Ernst, Ingo, D-6392 Neu-Anspach (DE); WIESNER, Matthias, D-6500 Mainz (DE); SCHLINGMANN, Merten, D-6240 Königstein (DE)
(86) Internationale Anmeldenummer: EP9000760
(87) Internationale Veröffentlichungsnummer: WO9014350

(56) Entgegenhaltungen:
- EP-A- 0 168 723
- EP-A- 0 212 374
- EP-A- 0 218 150
- EP-A- 0 298 438
- GB-A- 679 776

## Beschreibung

Die vorliegende Erfindung betrifft ein Verfahren zur Herstellung von 1-Fluor-glycuronsäuren (= 1-Fluor-1-desoxy-glycopyranuronsäuren= Glycopyranuronosylfluoriden) durch katalytische Oxidation der entsprechenden Glycosylfluoride und deren Umwandlung in die entsprechenden Salze. Sie betrifft ferner neue 1-Fluor-glycuronsäuren und deren Salze.

Da bei Aldosen die Aldehydgruppe sehr leicht zur Carboxylfunktion oxydiert wird, muß zur Synthese von Uronsäuren das anomere Zentrum (die Halbacetalgruppe) geschützt werden, gewöhnlich als Glycosid. Es ist bekannt, solche Glycoside mit Sauerstoff in Gegenwart von Platin/Kohle zu Glycuronsäure zu oxydieren (US-PS 2 562 200). Die Glycosidbindung wird, nachdem die primäre OH-Gruppe zur Carbonsäure oxydiert ist, sauer gespalten, wobei es wegen der drastischen Reaktionsbedingungen zu erheblichen Ausbeuteverlusten kommen kann (S.A.Barker, E.J.Bourne, M.Stacy, Chem. and Ind. 1951, 970). Erst dann ist eine Aktivierung und Verwendung der Uronsäuren z.B. für Glycosylierungsreaktionen möglich. Aus den alternativ eingesetzten Acetalen,z.B. O¹,O²-Isopropyliden-α-D-glucofuranose (C.L.Mehltretter, B.H.Alexander, R.L.Mellis, C.B.Rist, J.Am.Chem.Soc. 73, 2424 (1951)) oder O¹,O²-Cyclohexyliden-α-D-glucofuranose (C.L.Mehltretter, Adv. Carbohydr. Chem. 8, 244 (1953)), kann man zwar die durch Oxydation erhaltenen Produkt einfach und glatt die freien Uronsäuren gewinnen, jedoch sind diese Acetate wesentlich umständlicher und aufwendiger herzustellen.

Die Isolierung von Uronsäuren aus Naturstoffen ist oft mit erheblichen Reinigungsschritten und geringen Ausbeuten verbunden (R.L.Whistler, M.L.Wolfrom, Meth. Carbohydr. Chem. 2, 27 (1963)).

Überraschend wurde nun gefunden, daß die Bindung des Fluoratoms von Glycosylfluoriden unter den Bedingungen der katalytischen Oxydation mit Sauerstoff in Gegenwart von Edelmetallkatalysatoren in neutraler Lösung erstaunlich stabil ist, so daß sich solche Glycosylfluoride ohne eine Hydrolyse der C-F-Bindung in die entsprechenden 1-Fluor-glycuronsäuren überführen lassen.

Gegenstand der Erfindung ist nun ein Verfahren zur Herstellung von 1-Fluor-glycuronsäuren, die geschützte Aminogruppen enthalten können, und deren Salzen, das dadurch gekennzeichnet ist, daß man Glycopyranosylfluoride von Mono- bzw. Oligosacchariden, die wenigstens eine primäre OH-Funktion tragen, in wäßriger Lösung im pH-Bereich von 6 bis 9 mit Sauerstoff als Oxydationsmittel in Gegenwart eines Katalysators, der wenigstens ein Platinmetall enthält, oxydiert und die entstehenden 1-Fluorglycuronsäuren wenigstens teilweise neutralisiert.

Durch die Erfindung werden gleich mehrere Vorteile gegenüber den herkömmlichen Verfahrensweisen erreicht: Die 1-Fluor-glycuronsäure kann unter geeigneten Bedingungen, wie sie z.B. in den EP-OS 168723 = US-PS 4 749 785 und EP-OS 298438 beschrieben sind, direkt als Glycosyldonator in Glycosidierungsreaktionen oder enzymatischen Synthesen eingesetzt werden. Auch kann die Hydrolyse zur freien Glycuronsäure in saurem Medium rasch und ohne Nebenreaktionen vollzogen werden. Durch die Oxydation mit Sauerstoff vermeidet man zudem die bei anderen Oxydationsmethoden oft erheblichen Nachteile, wie etwa die zwangsläufige Bildung von unerwünschten Nebenprodukten - wie nitrosen Gasen oder Schwefelverbindungen - deren Entsorgung einen beträchtlichen Aufwand erfordert, oder die schwierige Abtrennung überschüssigen Oxydationsmittels. Bei der erfindungsgemäßen Oxydation entsteht dagegen neben den gewünschten Produkten zwangsweise nur noch Wasser, das ohnehin als Lösungsmittel verwendet wird.

Als Glycosylfluoride eignen sich die Fluoride von Mono- und Oligosacchariden, die wenigstens eine primäre OH-Funktion tragen, z.B. α-D-Glucopyranosylfluorid, α-D-Galactopyranosylfluorid, α-D-Mannopyranosylfluorid, α-D-Lactosylfluorid, α-D-Cellobiosylfluorid, α-D-Maltobiosylfluorid, Maltotriosylfluorid, Maltotetrosylfluorid u.a. Auch Glycosylfluoride solcher Saccharide, die noch Aminogruppen enthalten (die allerdings in geeigneter Weise geschützt sein müssen, z.B. durch Acyl-, Alkyl-, Aryl- oder Aralkylreste mit bis zu 10 C-Atomen) sind geeignet, vor allem solche mit bis zu 2 solcher Aminogruppen, wie das 2-Acetamido-2-desoxy-α-D-glucopyranosylfluorid. Wenngleich auch Glycosylfluoride oxydiert werden können, die zum Teil oder ganz an den sekundären OH-Gruppen geschützt sind, werden vorzugsweise ungeschützte Glycosylfluoride eingesetzt.

Es ist ein weiterer Vorteil des erfindungsgemäßen Verfahrens, daß man durch die Verwendung von Glycosylfluoriden ungeschützter oder an den Aminogruppen partiell geschützter Kohlenhydrate als Ausgangsstoffen die aufwendige Einführung von Schutzgruppen und deren spätere Wiederabspaltung vermeiden kann.

Als Katalysatoren eignen sich solche, die Platinmetalle, also Osmium, Iridium, Rhodium, Ruthenium, Palladium und/oder Platin, enthalten. Bevorzugt sind Katalysatoren, die eine Kombination von Palladium und Platin und insbesondere nur Platin enthalten. Vorzugsweise sind die Platinmetalle auf einem Träger, wie Al₂O₃ oder SiO₂, insbesondere auf Aktivkohle aufgebracht. Der Metallgehalt des Katalysators liegt im allgemeinen bei 1 bis 15, vorzugsweise bei 5 bis 10 Gew.-%.

Zuweilen kann es zweckmäßig sein, insbesondere wenn man geschützte Glycosylfluoride mit schlechterer Wasserlöslichkeit als Ausgangsstoffe verwendet, einen unter den Reaktionsbedingungen inerten Lösungsvermittler, vorzugsweise in einer Konzentration von 10 bis 75 Gew.-%, insbesondere 30 bis 50 Gew.-%, bezogen auf die Menge an Wasser und Lösungsvermittler, zuzusetzen. Geeignet sind vor allem solche Lösungsvermittler, die beim Durchleiten von Sauerstoff durch die wäßrige Lösung wenig flüchtig sind, so daß eine Explosionsgefahr im Dampfraum vermieden wird; auf der anderen Seite sollte der Lösungsvermittler nach der Oxydation leicht abtrennbar sein, beispielsweise durch Destillation. Geeignete Lösungsvermittler sind beispielsweise Glykoläther ohne freie OH-Gruppen, wie solche der Formel R¹O(CHRCH₂O)ₙR², wobei n eine Zahl von 1 bis 4, R H oder CH₃ und R¹ und R² jeweils unabhängig voneinander C₁-C₄-Alkyl bedeuten. Besonders geeignet sind die Dimethyl, Diäthyl- und Methyl-äthyl-äther der genannten allgemeinen Formel mit Siedepunkten im Bereich von 100 bis etwa 250°C, beispielsweise Di- und Triäthylenglykoldimethyl- und diäthyläther, Dipropylenglykoldimethyl- und -diäthyläther wovon Diäthylenglykoldimethyläther bevorzugt ist.

Die wäßrige Lösung enthält am Beginn der Oxydation zweckmäßig 5 bis 30 Gew.-%, vorzugsweise 10 bis 20 Gew.-%, bezogen auf die Summe von Wasser und Lösungsvermittler, an Glycosylfluorid.

Bevorzugtes Oxidationsmittel ist handelsüblicher (technisch reiner Sauerstoff. Es können jedoch auch Mischungen von Sauerstoff mit unter den Reaktionsbedingungen inerten Gasen, beispielsweise Mischungen von Sauerstoff mit Inertgasen, verwendet werden. Natürlich ist auch Luft selbst geeignet.

In der Regel arbeitet man bei einem Gesamtdruck von 0,5 bis 100 bar. Bei steigendem Druck steigt die Reaktionsgeschwindigkeit mit steigendem Sauerstoffpartialdruck deutlich an; jedoch kann der Vorteil der höheren Reaktionsgeschwindigkeit durch den bei Anwendung von höherem Druck erforderlichen höheren apparativen Aufwand in Bezug auf die Wirtschaftlichkeit überkompensiert werden. Bevorzugt ist ein Druckbereich von Atmosphärendruck bis 10 bar (absolut), wobei das Arbeiten bei Atmosphärendruck besonders einfach auszuführen ist.

Das erfindungsgemäße Verfahren wird in der Regel bei einer Temperatur von 5°C bis 80°C, vorzugsweise von 10°C bis 60°C, insbesondere von 20 bis 40°C durchgeführt.

Die Reaktion muß im etwa neutralen bis schwach alkalischen Medium, also von pH 6 bis 9, vorzugsweise von 6,5 bis 8,5 und insbesondere von 7 bis 8, durchgeführt werden, da die Glycosylfluoride in saurem Bereich nicht beständig sind. Die während der Oxydation entstehenden Carbonsäuren müssen daher abgefangen werden, z.B. durch geeignete Puffersubstanzen oder vorteilhaft durch Zugabe von wäßrigen Basen, etwa Alkali- oder Erdalkalihydroxyd-Lösungen, wobei diese so zudosiert werden, daß der pH-Wert des Reaktionssystems während der Oxydation im Bereich von 6 bis 9 bleibt. Bei einer vollständigen Neutralisation fallen die Oxydationsprodukte dann als Salze an.

Das erfindungsgemäße Verfahren kann in allen Apparaturen, die sich für die Durchführung von Reaktionen in der flüssigen Phase mit oder ohne Anwendung von Überdruck eignen, durchgeführt werden. Beispiele dafür sind die Durchführung in einem Rührkessel oder in einer Blasensäule mit suspendiertem Katalysator. Die Oxydation kann aber auch an einem Festbett mit gekörntem Katalysator in einem Rieselphasenreaktor durchgeführt werden.

Die erforderliche Reaktionszeit wird zweckmäßig dadurch ermittelt, daß man in gewissen Zeitabständen Proben der Reaktionslösung entnimmt und analysiert. Beispielsweise kann die Ausbeute der Reaktionsprodukte auf einfache Weise durch Analyse einer Probe mit Hilfe der Hochdruckflüssigkeitschromatographie im Vergleich zu Standardlösungen laufend bestimmt werden. Die Optimierung der Reaktionszeit ist zu empfehlen, da eine unnötig verlängerte Einleitung von Sauerstoff zu Überoxydationen und damit beispielsweise zu Decarboxylierungen und zur Verminderung der Ausbeute an den gewünschten Reaktionsprodukten führen kann.

Das Reaktionsgemisch kann nach üblichen Methoden aufgearbeitet werden. Z.B. werden zunächst das Wasser und etwa vorhandene Lösungsvermittler destillativ entfernt. Anschließend wird eine Reinigung, z.B. durch Chromatographie, Kristallisation oder Fällung, vorgenommen. Auch eine Abtrennung aus der bei der Oxydation erhaltenen Lösung über basische Ionenaustauscher in der OH⁻-Form ist möglich.

Das erfindungsgemäße Verfahren besitzt gegenüber den anfangs genannten herkömmlichen Methoden den weiteren Vorteil, daß am anomeren Zentrum keine Schutzgruppen eingeführt werden und sich die Produkte direkt als aktivierte Kohlenhydrate nutzen lassen.

Gegenstand der Erfindung sind auch Verbindungen der allgemeinen Formel I 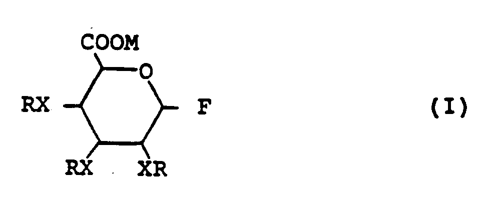
in, der
- M: für ein Kation, wie das des Li, Na, K, Mg und Ca, oder H oder NH₄, insbesondere aber für H oder ein Alkalimetallkation steht und
- X: unabhängig voneinander Sauerstoff oder NH bedeutet, wobei für X = Sauerstoff R die Bedeutung von Wasserstoff oder einem glycosidisch gebundenen Monosaccharid hat und für X = NH R für einen Acyl-, Alkyl-, Aryl- oder Aralkyl-Schutzgruppenrest mit bis zu 10 C-Atomen steht, wobei aber nur eine der Gruppen RX eine andere Bedeutung als OH hat.

Die 1-Fluor-glycuronsäuren sind wertvolle Ausgangsstoffe zur Herstellung von biologisch aktiven Di- und Oligosacchariden, die z.B. als Bestandteile von Zelloberflächen-Sacchariden und Glycoproteinen biologische Aktivität haben können, z.B. zur Stimulierung von Immunreaktionen und Antikörperbildung. Auch können, z.B. durch einfache Glycosylierungsreaktionen, oberflächenaktive Derivate erhalten oder die Lösungs- und Transporteigenschaften von Natur- und Wirkstoffen verbessert werden.

### Beispiele

1) In ein von außen beheizbares senkrecht angeordnetes Glasrohr (Durchmesser 25 mm, Länge 600 mm), das mit einer Mischung aus 10 g α-D-Glucopyranosylfluorid, 90 g Wasser und 5 g eines handelsüblichen Katalysators (5 Gew.-% Platin auf Aktivkohle, F 196 RAW der Fa.Degussa, Frankfurt, Deutschland) gefüllt war, leitete man von unten durch eine Glasfritte pro Stunde 10 Normal-Liter Sauerstoff bei einer Temperatur von 30°C ein. Durch kontinuierliche Zugabe von 30 %iger wäßriger Natronlauge wurde der pH-Wert bei 7 bis 7,5 gehalten. Nach 6 Stunden enthielt die Lösung 10,2 g Natrium-α-D-glucopyranuronatosylfluorid (85 % der Theorie). Das Produkt kann nach üblichen Verfahren isoliert und gereinigt werden.
   Weißes, amorphes Pulver; (α)_{D}: +71,2° (c = 1,08, Wasser)
   ¹H-NMR (D₂O): δ = 5,71 (dd, H-1), 3,67 (ddd, H-2), 3,77 (dd, H-3), 3,58 (dd, H-4), 4,09 (d, H-5); J_{1,F} = 53,4, J_{1,2}= 2,9, J_{2,F}= 26,6, J_{2,3}= 9,8, J_{3,4}= 9,3, J_{4,5}= 10,2 Hz.
   Massenspektroskopie (MS) (nach Silylierung): M⁺ = 484 (C₁₈H₄₁FO₆Si₄, 4fach silyliert)
2) Unter den in Beispiel 1 beschriebenen Bedingungen wurden 10 g α-D-Galactopyranosylfluorid in einer 10 %igen wäßrigen Lösung in Gegenwart von 5 g des dort genannten Katalysators bei 10°C mit Sauerstoff oxydiert. Nach 4 Stunden enthielt das Reaktionsgemisch 9,3 g Natrium-α-D-galactopyranuronatosylfluorid (78 % der Theorie), das nach üblichen Verfahren isoliert und gereinigt werden kann.
   Weißes, amorphes Pulver; (α)_{D}: +37,1° (c = 0,98, Wasser)
   ¹H-NMR (D₂O): δ = 5,73 (dd, H-1), 4,05 (mc, 4 H-2,3,4,5); J_{1,F}= 53,7, J_{1,2}= 2,2 Hz.
   MS (nach Silylierung): M⁺ = 484 (C₁₈H₄₁FO₆Si₄, 4fach silyliert)
3) In der in Beispiel 1 beschriebenen Vorrichtung wurde ein Gemisch aus 10 g 2-Acetamido-2-desoxy-α-D-glucopyranosylfluorid, 90 g Wasser und 5 g des dort genannten Katalysators bei 40°C mit Sauerstoff umgesetzt. Der pH-Wert wurde durch kontinuierliche Zugabe von 30 %iger Natronlauge auf 7 bis 7,5 gehalten. Nach 6 Stunden enthielt die Lösung 7,4 g Natrium-2-acetamido-2-desoxy-α-D-glucopyranuronatosylfluorid, entsprechend einer Ausbeute von, 70 % der Theorie, das isoliert und gereinigt werden kann.
   Weißes, amorphes Pulver; (α)_{D}: +23,5° (c = 1,14, Wasser)
   ¹H-NMR (D₂O): δ = 5,67 (dd, H-1), 4,06 (ddd, H-2), 3,81 (dd, H-3), 3,67 (dd, H-4), 4,11 (d, H-5), 2,07 (s, CH₃); J_{1,F} = 52,9, J_{1,2}= 3,0, J_{2,F}= 27,8, J_{2,3}= 9,9, J_{3,4}= 9,1, J_{4,5}= 10,0 Hz.
   MS (nach Silylierung): M⁺ = 453 (C₁₇H₃₆FNO₆Si₃, 3fach silyliert).
4) In der in Beispiel 1 beschriebenen Vorrichtung wurde ein Gemisch aus 10 g α-Lactosylfluorid (4-0-(β-D-Galactopyranosyl)-α-D-glucopyranosylfluorid), 90 g Wasser und 5 g des dort genannten Katalysators bei 30°C mit Sauerstoff umgesetzt. Der pH-Wert wurde durch kontinuierliche Zugabe von 30 %iger Natronlauge auf 7 bis 7,5 gehalten. Nach 5 Stunden enthielt die Lösung neben den beiden Monocarbonsäuren 4,9 g 1-Fluor-lactodiuronsäuressalz (Dinatrium 4-0-(β-D-galactopyranuronatosyl)-α-D-glucopyranuronatosylfluorid), entsprechend einer Ausbeute von 45 % der Theorie, das isoliert und gereinigt werden kann.
   Farbloser Sirup
   ¹H-NMR (D₂O): δ = 5,65 (dd, H-1); J_{1,2}= 2,7, J_{1,F}= 53,2 Hz.
   MS (nach Silylierung): M⁺ = 876 (C₃₃H₁₃FO₁₂Si₇, 7fach silyliert).

## Patentansprüche

1. Verfahren zur Herstellung von 1-Fluor-glycuronsäuren, die geschützte Aminogruppen enthalten können, und deren Salzen, dadurch gekennzeichnet, daß man Glycopyranosylfluoride von Mono- bzw. Oligosacchariden, die wenigstens eine primäre OH-Funktion tragen, in wäßriger Lösung im pH-Bereich von 6 bis 9 mit Sauerstoff als Oxydationsmittel in Gegenwart eines Katalysators, der wenigstens ein Platinmetall enthält, oxydiert und die entstehenden 1-Fluorglycuronsäuren wenigstens teilweise neutralisiert.

2. Verfahren nach Anspruch 1, dadurch gekennzeichnet, daß als Glycosylfluoride solche in ungeschützter Form eingesetzt werden.

3. Verfahren nach Anspruch 1 oder 2, dadurch gekennzeichnet, daß der Katalysator als Platinmetall eine Kombination von Palladium und Platin, insbesondere nur Platin enthält.

4. Verfahren nach einem oder mehreren der Ansprüche 1 bis 3, dadurch gekennzeichnet, daß der Katalysator aus 1 bis 15 Gew.-%, vorzugsweise 5 bis 10 Gew.-%, des Platinmetalls und einem Träger, vorzugsweise Aktivkohle, besteht.

5. Verfahren nach einem oder mehreren der Ansprüche 1 bis 4, dadurch gekennzeichnet, daß die Oxydation in einem Druckbereich von 0,5 bis 100 bar, vorzugsweise von Atmosphärendruck bis 10 bar, insbesondere bei Atmosphärendruck, und vorzugsweise mit technisch reinem Sauerstoff durchgeführt wird.

6. Verfahren nach einem oder mehreren der Ansprüche 1 bis 5, dadurch gekennzeichnet, daß die wäßrige Lösung einen unter den Reaktionsbedingungen inerten Lösungsvermittler, vorzugsweise in einer Menge von 10 bis 75 Gew.-%, insbesondere 30 bis 50 Gew.-%, enthält, wobei der Lösungsvermittler vorzugsweise ein Glykoläther ohne Hydroxygruppen, insbesondere Diäthylenglykoldimethyläther ist.

7. Verfahren nach einem oder mehreren der Ansprüche 1 bis 6, dadurch gekennzeichnet, daß die Oxydation bei einer Temperatur von 5 bis 80°C, vorzugsweise von 10 bis 60°C, insbesondere von 20 bis 40°C, durchgeführt wird.

8. Verfahren nach einem oder mehreren der Ansprüche 1 bis 7, dadurch gekennzeichnet, daß die wäßrige Lösung am Beginn der Oxydation 5 bis 30 Gew.-%, vorzugsweise 10 bis 20 Gew.-%, bezogen auf die Summe von Wasser und Lösungsvermittler, an Glycosylfluorid enthält.

9. Verfahren nach einem oder mehreren der Ansprüche 1 bis 8, dadurch gekennzeichnet, daß die Oxydation bei einem pH-Wert von 6,5 bis 8,5, vorzugsweise 7 bis 8 durchgeführt wird.

10. Verbindungen der allgemeinen Formel 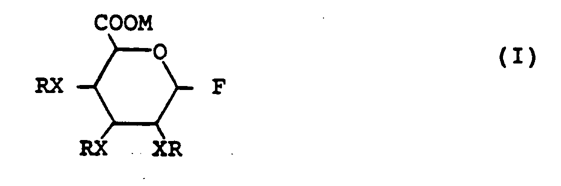 in der
M für Wasserstoff oder ein Kation, insbesondere Alkalimetall steht und
X unabhängig voneinander Sauerstoff oder NH bedeutet, wobei für X = Sauerstoff R die Bedeutung von Wasserstoff oder einem glycosidisch gebundenen Monosaccharid und für X = NH R für einen Acyl-, Alkyl-, Aryl- oder Aralkyl-Schutzgruppenrest mit bis zu 10 C-Atomen steht, wobei aber nur eine der Gruppen RX eine andere Bedeutung als OH hat.

## Claims

1. Process for the preparation of 1-fluoro-glycuronic acids, which can contain protected amino groups, and salts thereof, characterized in that glycopyranosyl fluorides of mono- or oligosaccharides, which carry at least one primary OH function, are oxidized with oxygen as the oxidizing agent in an aqueous solution in the pH range from 6 to 9 in the presence of a catalyst which contains at least one platinum metal, and the 1-fluoroglycuronic acids formed are at least partly neutralized.

2. Process according to Claim 1, characterized in that the glycosyl fluorides employed are glycosyl fluorides in unprotected form.

3. Process according to Claim 1 or 2, characterized in that the catalyst contains as platinum metal a combination of palladium and platinum, in particular only platinum.

4. Process according to one or more of Claims 1 to 3, characterized in that the catalyst comprises 1 to 15 % by weight, preferably 5 to 10 % by weight, of the platinum metal and a support, preferably active charcoal.

5. Process according to one or more of Claims 1 to 4, characterized in that the oxidation is carried out in a pressure range from 0.5 to 100 bar, preferably atmospheric pressure to 10 bar, in particular at atmospheric pressure, and preferably using industrially pure oxygen.

6. Process according to one or more of Claims 1 to 5, characterized in that the aqueous solution contains a solubilizing agent which is inert under the reaction conditions, preferably in an amount of 10 to 75 % by weight, in particular 30 to 50 % by weight, the solubilizing agent preferably being a glycol ether containing no hydroxyl groups, in particular diethylene glycol dimethyl ether.

7. Process according to one or more of Claims 1 to 6, characterized in that the oxidation is carried out at a temperature of 5 to 80°C, preferably 10 to 60°C, in particular 20 to 40°C.

8. Process according to one or more of Claims 1 to 7, characterized in that, at the start of the oxidation, the aqueous solution contains 5 to 30 % by weight, preferably 10 to 20 % by weight, based on the sum of water and solubilizing agent, of glycosyl fluoride.

9. Process according to one or more of Claims 1 to 8, characterized in that the oxidation is carried out at a pH of 6.5 to 8.5, preferably 7 to 8.

10. Compounds of the general formula 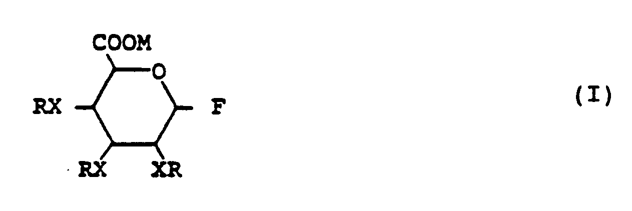 in which
M represents hydrogen or a cation, in particular an alkali metal, and
X denotes oxygen or NH, independently at each occurrence, where, if X = oxygen, R denotes hydrogen or a glycosidically bonded monosaccharide and, if X = NH, R represents an acyl, alkyl, aryl or aralkyl protective group radical having up to 10 carbon atoms, but where only one of the groups RX has a meaning other than OH.

## Revendications

1. Procédé pour la préparation d'acides 1-fluoro-glycuroniques qui peuvent contenir des groupes amino protégés, et de leurs sels, caractérisé en ce qu'on oxyde des fluorures de glycopyranosyle des mono-, respectivement oligosaccharides, qui portent au moins une fonction OH primaire, en solution aqueuse dans un domaine de pH de 6 à 9, par l'oxygène en tant qu'agent oxydant, en présence d'un catalyseur qui contient au moins un métal de la famille du platine, et on neutralise, au moins partiellement, les acides 1-fluoro-glycuroniques formés.

2. Procédé selon la revendication 1, caractérisé en ce qu'on utilise en tant que fluorures de glycosyle ceux qui sont sous forme non protégée.

3. Procédé selon la revendication 1 ou 2, caractérisé en ce que le catalyseur contient en tant que métal de la famille du platine une combinaison de palladium et de platine, plus particulièrement seulement du platine.

4. Procédé selon une ou plusieurs des revendications 1 à 3, caractérisé en ce que le catalyseur se compose de 1 à 15 % en poids, de préférence de 5 à 10 % en poids, du platine métal et d'un support, de préférence le charbon actif.

5. Procédé selon une ou plusieurs des revendications 1 à 4, caractérisé en ce qu'on met en oeuvre l'oxydation dans un domaine de pressions de 0,5 à 100 bars, de préférence de la pression atmosphérique à 10 bars, plus particulièrement à la pression atmosphérique, et de préférence avec l'oxygène de pureté technique.

6. Procédé selon une ou plusieurs des revendications 1 à 5, caractérisé en ce que la solution aqueuse contient dans les conditions de réaction un agent solubilisant inerte, de préférence en une quantité de 10 à 75 % en poids, plus particulièrement de 30 à 50 % en poids, l'agent solubilisant étant de préférence un éther de glycol sans groupes hydroxy, plus particulièrement l'éther diméthylique du diéthylène-glycol.

7. Procédé selon une ou plusieurs des revendications 1 à 6, caractérisé en ce qu'on met en oeuvre l'oxydation à une température de 5°C à 8°C, de préférence de 10°C à 6°C, plus particulièrement de 20°C à 4°C.

8. Procédé selon une ou plusieurs des revendications 1 à 7, caractérisé en ce que la solution aqueuse contient au début de l'oxydation de 5 à 30 % en poids, de préférence de 10 à 20 % en poids, par rapport à la somme d'eau et d'agent solubilisant, de fluorure de glycosyle.

9. Procédé selon une ou plusieurs des revendications 1 à 8, caractérisé en ce qu'on met en oeuvre l'oxydation à une valeur de pH de 6,5 à 8,5, de préférence de 7 à 8.

10. Composé de formule générale (I) 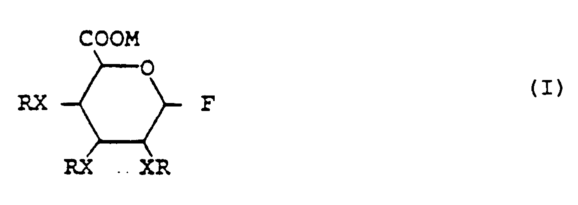 dans laquelle
M représente un cation, plus particulièrement un cation de métal alcalin et
les radicaux X représentent indépendamment l'un de l'autre l'oxygène ou NH, pour X = oxygène, R ayant la signification de l'hydrogène ou d'un monosaccharide lié comme glycoside, et pour X = NH R représentant un radical d'un groupe protecteur acyle, alkyle, aryle ou aralkyle comportant jusqu'à 10 atomes de carbone, mais seulement un des groupes RX ayant une signification autre que OH.
